# EUROPEAN PATENT SPECIFICATION

(11) **EP 4 123 011 B1**
(45) Date of publication and mention of the grant of the patent: **06.05.2026**
(21) Application number: 21771082.1
(22) Date of filing: 18.03.2021
(51) Int. Cl.: C12N 5/00, C12M 3/00

(54) **AGENT FOR PROMOTING SPHEROID FORMATION**
MITTEL ZUR FÖRDERUNG DER SPHÄROIDBILDUNG
AGENT FAVORISANT LA FORMATION DE SPHÉROÏDES

(30) Priority: 19.03.2020 JP 2020049062
(43) Date of publication of application: 25.01.2023
(73) Proprietor: Kunimine Industries Co., Ltd., Chiyoda-ku Tokyo 101-0032 (JP); OSAKA UNIVERSITY, Suita-shi Osaka 565-0871 (JP)
(72) Inventor: KUBOTA, Munehiro, Iwaki-shi, Fukushima 972-8312 (JP); TSUJIKAWA, Kazutake, Suita-shi, Osaka 565-0871 (JP)
(74) Representative: Forstmeyer, Dietmar
(86) International application number: PCT/JP2021/011128
(87) International publication number: WO 2021/187566

(56) References cited:
- JP-A- 2010 018 775
- JP-A- 2012 515 001
- JP-A- 2017 060 457
- JP-A- 2019 163 212
- JP-A- 2019 163 212
- JP-A- H0 380 075
- KR-A- 20190 053 006
- US-A- 3 935 069
- JACKLYN WHITEHEAD ET AL: "Morphogen Delivery by Osteoconductive Nanoparticles Instructs Stromal Cell Spheroid Phenotype", ADVANCED BIOSYSTEMS, JOHN WILEY & SONS, INC, HOBOKEN, USA, vol. 3, no. 12, 1 October 2019 (2019-10-01), pages n/a, XP072279632, ISSN: 2366-7478, DOI: 10.1002/ADBI.201900141
- TAGAYA MOTOHIRO ET AL: "Nano/Microstructural Effect of Hydroxyapatite Nanocrystals on Hepatocyte Cell Aggregation and Adhesion", MACROMOLECULAR BIOSCIENCE, vol. 11, no. 11, 25 August 2011 (2011-08-25), DE, pages 1586 - 1593, XP093139845, ISSN: 1616-5187, DOI: 10.1002/mabi.201100182
- WHITEHEAD JACKLYN, KOTHAMBAWALA ALEFIA, KENT LEACH J.: "Morphogen Delivery by Osteoconductive Nanoparticles Instructs Stromal Cell Spheroid Phenotype", ADVANCED BIOSYSTEMS, vol. 3, no. 12, 1 December 2019 (2019-12-01), pages 1900141, XP055859400, ISSN: 2366-7478, DOI: 10.1002/adbi.201900141

## Description

### TECHNICAL FIELD

The present invention relates to a method of producing animal cells in a spheroid form.

### BACKGROUND ART

In recent years, animal cells of mammalians represented by human beings have been used as research materials for a wide variety of studies.

In plane culture (two-dimensional cell culture) of animal cells, which has been performed for a long time, cells expand two-dimensionally and proliferate. Thus, a certain culture area is required to obtain a certain number of cultured cells. Further, since the cultured cells are monolayer cells spreading in a two-dimensional direction, there is a problem that the function of the cells themselves cannot necessarily reproduce the state in vivo.

Thus, three-dimensionally culturing cells utilizing an original aggregation reaction of adherent animal cells is widely desired. A spheroid (aggregate) obtained by three-dimensional cell culture exhibit a function different from that of cells obtained by two-dimensional cell culture, realize a cell environment closer to a living body, and have high cell activity. Thus, spheroids are useful for drug screening in drug discovery, toxicity tests, construction of a safety evaluation model as an alternative to animal experiments and the like, and are also very important as a biomaterial from which practical information can be obtained.

In general three-dimensional cell culture methods, control of the adhesion of cells is important, and scaffold materials, culture vessels, media and the like that are suitably used in three-dimensional cell culture methods have been developed.

For example, porous membranes and hydrogels used as scaffold materials are commercially available, and Patent Literature 1 describes a method of culturing cells using a chitin gel produced by gelating chitosan by treating the chitosan with an acetylating agent as a culture base material. Patent Literature 2 describes a base material for cell culture in which a chimeric protein having a hydrophobic material binding site and a cell adhesion site is immobilized on a fiber surface of a fiber structure having a specific average fiber diameter and formed of a polymer compound. Further, Non-Patent Literature 1 describes control of adhesion of cells using a hydrogel in which an inorganic layered compound is present in a polymer matrix.

Regarding culture vessels, a plate subjected to microfabrication for the purpose of non-adhesion treatment, a U-bottom plate designed to collect cells and the like can be used for three-dimensional cell culture methods, and are also commercially available.

Further, Patent Literature 3 describes that spheroid formation is promoted by culturing a mixture of a cell sample and a polymer obtained by polymerization of one or more selected from the group consisting of D-glucosamine, D-galactosamine, D-glucuronic acid, L-iduronic acid, and D-galactose in a vessel for spheroid formation culture. Patent Literature 4 describes a method of producing a spheroid including the step of culturing cells in the presence of an exogenous cell aggregating agent containing any of an antibody, a lectin, and a cell adhesion molecule.

As described above, three-dimensional cell culture can be performed by using special scaffold materials, culture vessels, or culture media. However, such scaffold materials, culture vessels, or culture media are generally expensive. Thus, when a large amount of spheroids is required for, for example, screening for drugs in drug discovery, screening for drug efficacy in primary culture of clinical specimens, and production of useful proteins, the cost burden is large.

Further, development of a three-dimensional cell culture method having a high spheroid formation rate and excellent versatility without limitation of target biological tissues or cell types is also desired.

A swellable layered silicate such as smectite is a kind of clay minerals, and has a property of swelling and becoming viscous when absorbing a liquid such as water. Smectite is composed of nanosheets spreading in two dimensions. When smectite is swollen with and dispersed in water, a colloidal dispersion liquid having viscosity and thixotropy is obtained. Smectite exists in a state of being separated up to a unit layer in a dispersion liquid. The crystal layer itself of smectite in the dispersion liquid has a permanent negative charge, and cations such as sodium ions are incorporated into the crystal layer to compensate for the permanent negative charge. By utilizing such properties, swellable layered silicates such as smectite have been industrially widely used since a long time ago.

For example, a protein crystal formation controlling agent containing a layered silicate compound containing a fluorine atom is described in Patent Literature 5. Further, an agent for searching for protein crystallization conditions, containing a water-swellable layered silicate having a fluorine atom and a hydroxyl group, wherein the fluorine atom is covalently bonded to the silicate by isomorphous substitution with the hydroxyl group is described in Patent Literature 6.

Patent Literature 7 describes using a body fluid collected from a site from an epidermis to a muscle tissue of a predetermined fish having a mucusy body epidermis, or a body fluid in a trunk portion or a body fluid collected from a liver of a cephalopod as a spheroid formation promoter. Patent Literature 8 describes a method of removing odor components and other contaminants of the spheroid formation promoter using a powder of aluminum silicate or natural allophane to concentrate or purify the spheroid formation promoter.

However, practical reports have not yet been made on the fact that inorganic particle materials themselves such as swellable layered silicates promote the formation of spheroids.

### CITATION LIST

### PATENT LITERATURES

Patent Literature 1: JP-A-2017-55727 ("JP-A" means an unexamined published Japanese patent application)
Patent Literature 2: JP-A-2010-63411
Patent Literature 3: JP-A-2017-147944
Patent Literature 4: JP-A-2008-22743
Patent Literature 5: JP-A-2011-121789
Patent Literature 6: WO 2012/133695
Patent Literature 7: JP-A-2011-62129
Patent Literature 8: JP-A-2019-163212

### NON-PATENT LITERATURES

Non Patent Literature 1: Advanced Materials, 2013, Volume 25, Issue 30, p. 4069-4086

KR 2019 0053006 A discloses hydroxyapatite microspheres. J. Whitehead et al. Adv. Biosys. 2019, 3, 1900141 relates to the aggregation of MSCs into spheroids to maintain osteogenic differentiation. M. Tagaya et al. Macromol. Biosci. 2011, 11, 1586-1593 relates to the effect of hydroxyapatite nanocrystals on hepatocyte cell aggregation and adhesion. US 3,935,069 discloses the preparation of flocculating microbial cell aggregates.

### SUMMARY OF INVENTION

### TECHNICAL PROBLEM

As described above, at present, use of special scaffold materials, culture vessels, or culture media is necessary in the production of spheroids. Further, in the production of spheroids, since the adhesion force inherent in cells is used as a driving force, it may take a long time to form spheroids.

Thus, in conventional methods, it has been difficult to easily and quickly perform spheroid formation for various cell types in a versatile manner.

The present invention provides a method of producing animal cells in a spheroid form.

### SOLUTION TO PROBLEM

In view of the above problems, the present inventors have conducted intensive studies. As a result, the present inventors have found that particles composed of specific inorganic substances such as a swellable layered silicate, silicates such as kaolinite, mica, and zeolites, colloidal silica, alumina hydrate, magnesium oxide, and hydroxyapatite have an action of promoting aggregation or organization of cells. Further, the present inventors have found that when three-dimensional cell culture is performed in a state where a predetermined amount of specific inorganic particles is dispersed in a cell suspension for spheroid formation, cells in a spheroid form can be obtained easily in a short period of time.

The present invention has been completed based on these findings.

In the present invention, the above problems were solved by the following means:
A method of producing animal cells in a spheroid form, comprising the steps of:
mixing a dispersion liquid of inorganic particles having adsorbability and a cell suspension to obtain a mixture in which the inorganic particles having adsorbability are dispersed, and
culturing a cell contained in the obtained mixture to form a spheroid,
wherein the solid content concentration of the adsorptive inorganic particles in the dispersion liquid is 0.001 mass% or more and less than 1.000 mass%,
wherein the inorganic particles act as spheroid formation promoter, and
wherein the inorganic particles are particles composed of at least a swellable layered silicate, wherein the solid content concentration of the swellable layered silicate in the mixture of the cell suspension and the dispersion liquid of the swellable layered silicate is 0.0003 w/v% or more and 0.1667 w/v% or less,
wherein an adsorption amount of bovine serum albumin per 1 mg of the inorganic particles having adsorbability is 10 µg or more and 500 µg or less.

Preferably, the inorganic particles are particles composed of at least one kind of swellable layered silicate selected from the group consisting of montmorillonite, hectorite, stevensite, saponite, beidellite, nontrite, sauconite, and swellable mica.

Further preferably, the animal cell is a cell collected from an animal, a cell obtained by culturing a cell collected from an animal, a cell obtained by subjecting a cell collected from an animal to various treatments, or a cultured cell line, and/or the cell is a mammalian animal cell.

Moreover preferably, the animal cell is cultured in a culture plate for suspension cells to form a spheroid.

### ADVANTAGEOUS EFFECTS OF INVENTION

According to the method of producing animal cells in a spheroid form of the present invention, a spheroid can be easily and quickly formed.

The above and other features and advantages of the present invention will become more apparent from the following description appropriately with reference to the attached drawings.

### BRIEF DESCRIPTION OF THE DRAWINGS

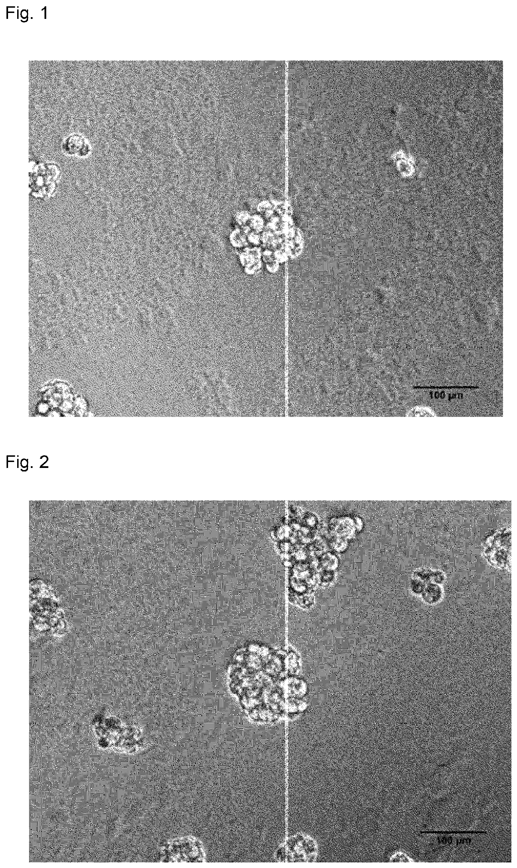

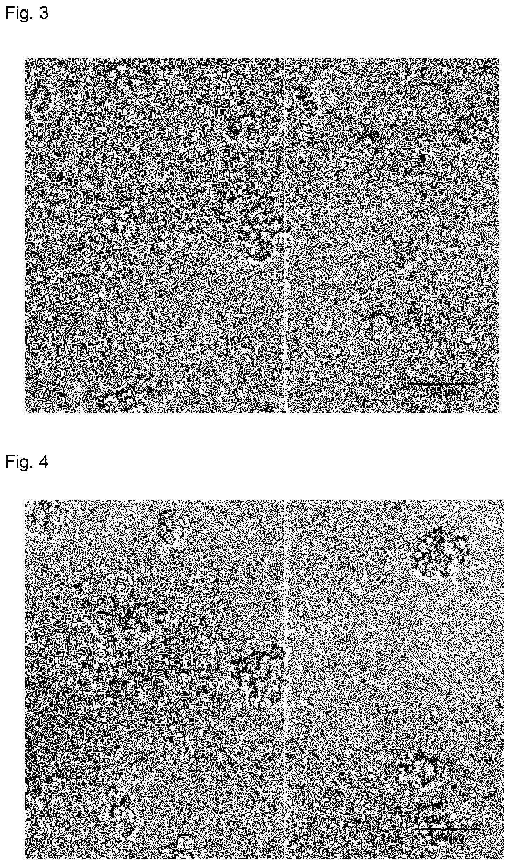

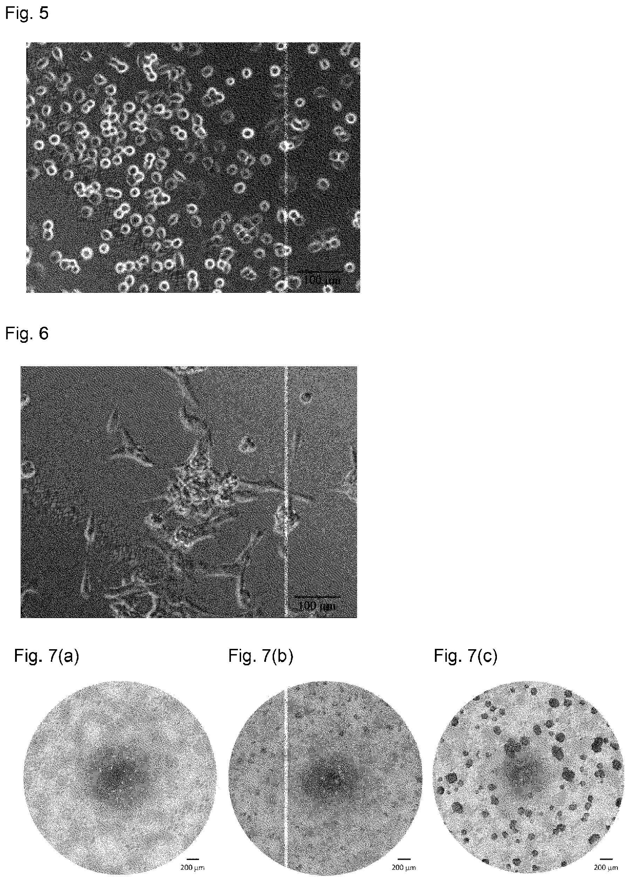

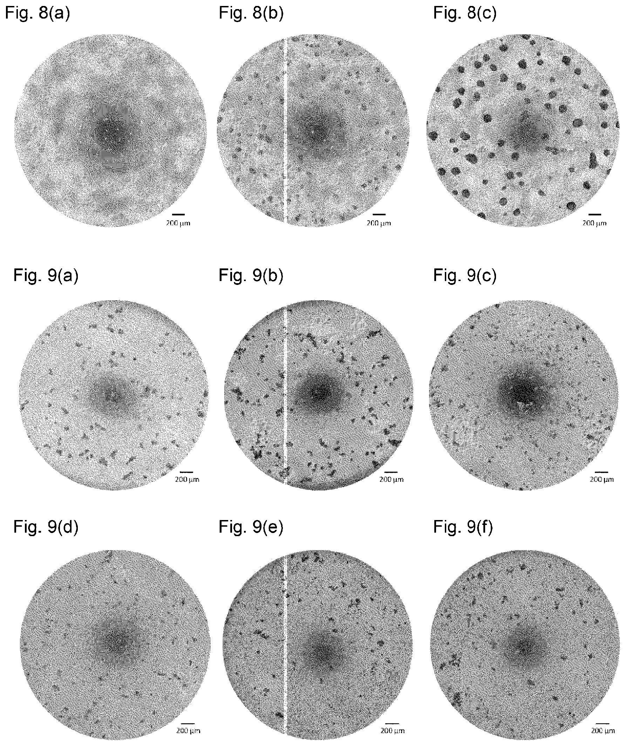

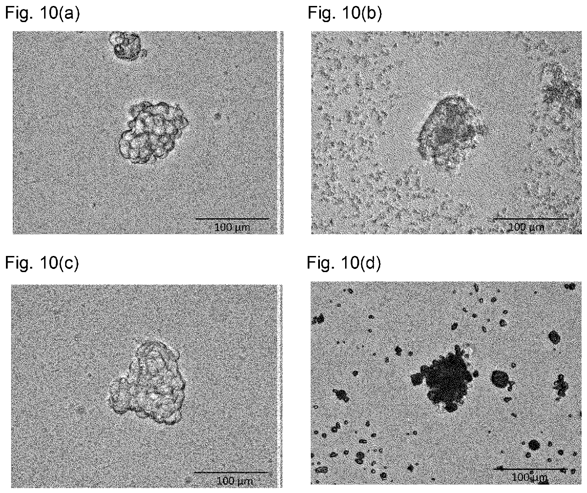

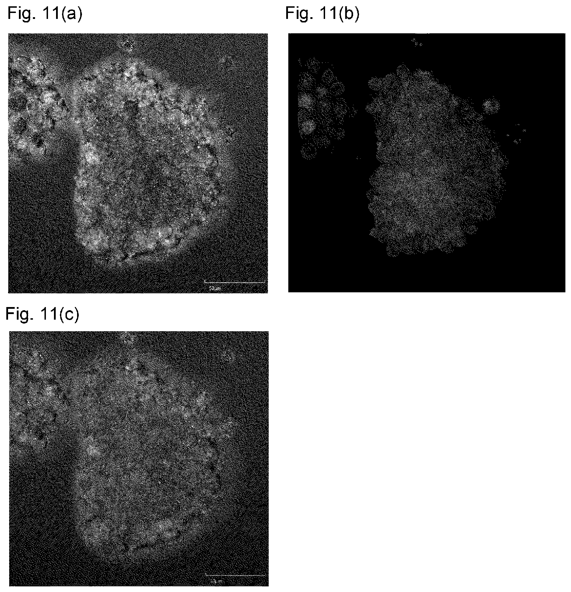

{Fig. 1}
   Fig. 1 is a photomicrograph of spheroids formed after culturing a human pancreatic cancer cell PANC-1 strain for 3 days in a mixture in which a swellable layered silicate (fluorinated sodium hectorite) has been dispersed so that the solid content concentration of the swellable layered silicate would be 0.0052 w/v%, using a culture plate for suspension cells.
{Fig. 2}
   Fig. 2 is a photomicrograph of spheroids formed after culturing a human pancreatic cancer cell PANC-1 strain for 3 days in a mixture in which a swellable layered silicate (sodium hectorite powder) has been dispersed so that the solid content concentration of the swellable layered silicate would be 0.0052 w/v%, using a culture plate for suspension cells.
{Fig. 3}
   Fig. 3 is a photomicrograph of spheroids formed after culturing a human kidney cancer cell ACHN strain for 3 days in a mixture in which a swellable layered silicate (fluorinated sodium hectorite) has been dispersed so that the solid content concentration of the swellable layered silicate would be 0.0052 w/v%, using a culture plate for suspension cells.
{Fig. 4}
   Fig. 4 is a photomicrograph of spheroids formed after culturing a human kidney cancer cell ACHN strain for 3 days in a mixture in which a swellable layered silicate (sodium hectorite powder) has been dispersed so that the solid content concentration of the swellable layered silicate would be 0.0052 w/v%, using a culture plate for suspension cells.
{Fig. 5}
   Fig. 5 is a photomicrograph showing the state of cells obtained after culturing a human pancreatic cancer cell PANC-1 strain for 3 days in a cell suspension not containing a swellable layered silicate.
{Fig. 6}
   Fig. 6 is a photomicrograph showing the state of cells obtained after culturing a human kidney cancer cell ACHN strain for 3 days in a cell suspension not containing a swellable layered silicate.
{Fig. 7}
   Figs. 7(a) to 7(c) are photomicrographs of spheroids formed after culturing a human pancreatic cancer cell PANC-1 strain in a mixture in which a swellable layered silicate (sodium hectorite) has been dispersed so that the solid content concentration of the swellable layered silicate would be 0.0052 w/v%, Fig. 7(a) is a photograph showing the state on Day 1 from the start of culture, Fig. 7(b) is a photograph showing the state on Day 3 from the start of culture, and Fig. 7(c) is a photograph showing the state on Day 7 from the start of culture.
{Fig. 8}
   Figs. 8(a) to 8(c) are photomicrographs of spheroids formed after culturing a human kidney cancer cell ACHN strain in a mixture in which a swellable layered silicate (sodium hectorite) has been dispersed so that the solid content concentration of the swellable layered silicate would be 0.0052 w/v%, Fig. 8(a) is a photograph showing the state on Day 1 from the start of culture, Fig. 8(b) is a photograph showing the state on Day 3 from the start of culture, and Fig. 8(c) is a photograph showing the state on Day 7 from the start of culture.
{Fig. 9}
   Figs. 9(a) to 9(f) are photomicrographs of spheroids after culturing human colon cancer cell HT29 for 4 days in the presence of various inorganic particles. Fig. 9(a) is a photomicrograph of spheroids in a mixture in which colloidal silica (trade name: SNOWTEX ST-C) has been dispersed so that the concentration would be 0.005 w/v%, Fig. 9(b) is a photomicrograph of spheroids in a mixture in which colloidal silica (trade name: SNOWTEX ST-CXS) has been dispersed so that the concentration would be 0.0025 w/v%, Fig. 9(c) is a photomicrograph of spheroids in a mixture in which alumina hydrate particles (trade name: Alumina Sol AS-200) has been dispersed so that the concentration would be 0.02 w/v%, Fig. 9(d) is a photomicrograph of spheroids in a mixture in which alumina hydrate particles (trade name: Alumina Sol AS-520-A) has been dispersed so that the concentration would be 0.08 w/v%, Fig. 9(e) is a photomicrograph of spheroids in a mixture in which phyllosilicate mineral particles (trade name: Kaolin JP-100) has been dispersed so that the concentration would be 0.02 w/v%, and Fig. 9(f) is a photomicrograph of spheroids in a mixture in which phyllosilicate mineral particles (trade name: MK-100) has been dispersed so that the concentration would be 0.04 w/v%.
{Fig. 10}
   Figs. 10(a) to 10(d) are photomicrographs of spheroids after culturing a human kidney cancer cell ACHN strain for 5 days in the presence of various inorganic particles. Fig. 10(a) is a photomicrograph of spheroids in a mixture in which tectosilicate mineral particles (trade name: Zeoal-ZSM-5) have been dispersed so that the concentration would be 0.02 w/v%, Fig. 10(b) is a photomicrograph of spheroids in a mixture in which tectosilicate mineral particles (trade name: HSZ-940NHA) have been dispersed so that the concentration would be 0.04 w/v%, Fig. 10(c) is a photomicrograph of spheroids in a mixture in which magnesium oxide particles (trade name: KYOWA MAG MF-150) have been dispersed so that the concentration would be 0.04 w/v%, and Fig. 9(d) is a photomicrograph of spheroids in a mixture in which hydroxyapatite particles (trade name: SHAp) have been dispersed so that the concentration would be 0.04 w/v%.
{Fig. 11}
   Figs. 11(a) to 11(c) are double-stained observation images of a spheroid obtained by adding a swellable layered silicate (sodium hectorite) having a fluorescent marker to a human colon cancer cell SW620 strain, and culturing the strain for 5 days, Fig. 11(a) is an observation image in a bright field, Fig. 11(b) is an observation image in a fluorescence field, and Fig. 11(c) is a synthetic image in which the bright field and the fluorescence field are superimposed.

### MODE FOR CARRYING OUT THE INVENTION

In the present invention, inorganic particles having adsorbability (hereinafter, also simply referred to as "adsorptive inorganic particles") are used as an active ingredient of a spheroid formation promoter.

As demonstrated in Examples described later, adsorptive inorganic particles exhibit an action of promoting aggregation or organization of cells. Further, by performing three-dimensional cell culture in a state where a predetermined amount of adsorptive inorganic particles is dispersed, spheroids are easily and quickly formed.

Hereinafter, the method of the present invention will be described based on preferable embodiments. However, the present invention is not limited thereto.

The "three-dimensional cell culture" in the present specification is a technique of culturing cells in an artificially produced environment while making cells three-dimensionally interact with the surrounding environment. Three-dimensional cell culture differs from two-dimensional cell culture and allows cells to grow in all directions in vitro, same as cells in vivo.

A minute mass of cells formed by gathering of cells utilizing the adhesion of specific cells by three-dimensional cell culture is a "spheroid".

The inorganic particles used in the present invention have adsorbability. In the present specification, the term "adsorbability" means adsorbability to proteins such as serum components and growth factors. Further, the inorganic particles used in the present invention or the composite particles in which a protein is adsorbed to inorganic particles are also adsorbed to individual surfaces of cells that form a spheroid, thereby promoting aggregation of cells to form a spheroid.

The adsorbability of the inorganic particles can be judged by an ordinarily method, and for example, the adsorption ability of the inorganic particles can be measured using a reagent of bovine serum albumin (hereinafter, also simply referred to as "BSA"). For example, a predetermined amount of a dispersion liquid of colloidal particles is added to a solution in which BSA is dissolved, then solid-liquid separation is performed by centrifugation, and the amount of BSA in the supernatant component is quantified. Since BSA to which inorganic particles are adsorbed moves to the solid component after centrifugation, the amount of BSA to which inorganic particles are adsorbed can be calculated by subtracting the amount of BSA in the supernatant component from the initial amount of BSA. In this way, the protein adsorption ability of the inorganic particles can be determined. Inorganic particles having protein adsorption ability are effective for the formation of spheroids, and can be used in the present invention.

The inorganic particles used in the present invention are particles composed of at least a swellable layered silicate.

In the present invention, the reason (mechanism) that the formation of spheroids is promoted by three-dimensional cell culture is not clear. Presumably, serum or a protein such as a growth factor contained in the culture solution of cells that form spheroids and the adsorptive inorganic particles are adsorbed to each other, and the resulting complex itself composed of the protein and the adsorptive inorganic particles is adsorbed to the surface of cells and acts as a scaffold, thereby promoting the formation of spheroids.

In particular, it is presumed that the particles composed of a swellable layered silicate are present in the medium as nanoscale flat plate particles, and thus the swellable layered silicate acts as a small scaffold in spheroid formation. Further, presumably, since the layer surface of the swellable layered silicate is negatively charged, the protein (serum component) in the medium is adsorbed to the swellable layered silicate, and the protein is supplied to the cell surface via the swellable layered silicate.

The "silicate" that can be used as an active ingredient of the spheroid formation promoter of the present invention refers to a compound containing an anion and having a structure in which one or several silicon atoms surrounded by electronegative ligands are at the center. In the majority of silicates, the silicon atom forms a tetrahedral structure surrounded by four oxygen atoms. Depending on the kind of silicates, the degree of connection of the tetrahedrons varies, and depending on the type of connection of the tetrahedrons, there are various structures such as a pair, a cluster, a ring, a chain, a double chain, a layer, and a three-dimensional network.

The "swellable layered silicate" that is used in the present invention refers to a compound that exhibits water swellability and has a layer structure in which a large number of two-dimensional unit layers are laid, the layer structure being composed of at least a silicon atom and electrically negative ligands. The layered silicate may have a single layer of a tetrahedron sheet and/or an octahedron sheet, or a mixed sheet thereof. A tetrahedron sheet takes a structure in which four oxygen ions (O²⁻) surround a silicon ion (Si⁴⁺) as a standard element, the three vertices are shared with adjacent tetrahedrons, and the resultant structure constitutes a hexagonal network, and is connected in a sheet form. An octahedron sheet has a two-dimensionally spread structure in which octahedrons formed by six oxygen ions (O²⁻) or hydroxide ions (OH⁻) surrounding a magnesium ion (Mg²⁺) or aluminum ion (Al³⁺) share edges. When the tetrahedron sheet and the octahedron sheet are combined, the oxygen ions at the vertices of the tetrahedron sheet are shared. Further, a part of the tetrahedron sheet is isomorphically substituted with an aluminum ion, and a part of the octahedron sheet is isomorphically substituted with an aluminum ion, a magnesium ion, an iron ion (Fe²⁺, Fe³⁺), a lithium ion (Li⁺) or the like, thereby negative charges are generated. Alternatively, the presence of voids in a part of the tetrahedron sheet and the octahedron sheet also generates negative charges. Cations exist between layers to neutralize these negative charges.

The swellable layered silicate has various properties such as swellability, thickening, thixotropy, and cation exchangeability. Further, since the swellable layered silicate is an inorganic substance, the swellable layered silicate is hardly decomposed or altered by microorganisms, and is also a material that is human body-friendly. Further, since the layered silicate used in the present invention has water swellability, the layered silicate is less likely to be naturally precipitated even in a cell suspension. Thus, a spheroid formation promoter that is excellent in uniform dispersibility and can stably maintain a uniform dispersion state for a long time can be provided.

The swellable layered silicate that can be used in the present invention is preferably derived from a mineral that belongs to the smectite group. The minerals that belong to the smectite group have a 2:1 layered structure. The primary particles of smectite are plate-like crystals having a thickness of 1 nm and a spread of 20 nm to 2 µm. As described above, in a water dispersion liquid, cations such as sodium ions are taken into the crystal layer to compensate for permanent negative charges of the smectite crystal layer itself (see "Clay Handbook", third edition, edited by The Clay Society of Japan, May 2009, p. 65.).

Specific examples of the swellable layered silicate that can be used in the present invention include montmorillonite, hectorite, stevensite, saponite, beidellite, nontrite, sauconite, and swellable mica.

The swellable layered silicate that can be used in the present invention may have any cation exchange capacity as long as the effect of the present invention is not impaired. The cation exchange capacity of the swellable layered silicate is preferably 10 meq/100 g or more, and more preferably 30 meq/100 g or more. The cation exchange capacity in a suitable range provides the swellable layered silicate with a negative charge suitable for adsorption of proteins (serum components). The swellable layered silicate that can be used in the present invention is preferably a monovalent cation silicate such as sodium from the viewpoint of achieving excellent swellability and dispersion stability, and more preferably a monovalent cation swellable layered silicate such as sodium from the viewpoint of achieving excellent swellability and dispersion stability. The cation exchange capacity of the swellable layered silicate can be measured by a method according to the Schollenberger method (Clay Handbook, 3rd edition, edited by The Clay Society of Japan, May 2009, p. 453-454). More specifically, it can be measured by the method described in Japan Bentonite Industry Association Standard Test Method JBAS-106-77. For example, the leaching cation amount of montmorillonite can be calculated by leaching interlayer cations of montmorillonite using 100 mL of a 1 M aqueous ammonium acetate solution with respect to 0.5 g of montmorillonite over 4 hours or more, and measuring the concentrations of various cations in the obtained solution by ICP emission analysis, atomic absorption analysis or the like. Though the upper limit of the cation exchange capacity of the swellable layered silicate that can be used in the present invention is not particularly limited, it is practically 120 meq/100 g or less.

As the silicate, in addition to the swellable layered silicate, a silicate mineral composed of a silicate can be used. The silicate mineral that can be used is not particularly limited, and examples thereof include nesosilicate minerals such as olivine group, garnet, zircon, sillimanite, andalusite, kyanite, titanite, topaz, staurolite, datolite, braunite, chloritoid, alleghanyite, spurrite, dumortierite, malayaite, dioptase, willemite, phenakite, knebelite, glaucochroite, humite, homilite, euclase, and gadolinite; sorosilicate minerals such as melilite, gehlenite, lawsonite, zoisite, clinozoisite, epidote, piemontite, allanite-(Ce), vesuvianite, idocrase, hemimorphite, ilvaite, lievrite, zunyite, and bertrandite; cyclosilicate minerals such as axinite group, beryl, cordierite, osumilite, sugilite, sogdianite, and tourmaline; inosilicate minerals such as pyroxene group, pyroxenoid group, pectolite, babingtonite, amphibole group, and plancheite; phyllosilicate minerals such as kaolinite, halloysite, serpentine, garnierite, pyrophyllite, talc, mica group, chlorite group, vermiculite, gyrolite, okenite, prehnite, apophyllite group, and chrysocolla; and tectosilicate minerals such as feldspar group, feldspathoid group, scapolite, zeolite group, leucite, ammonioleucite, inesite, danburite, helvite, and danalite. Among them, phyllosilicate minerals such as kaolinite, halloysite, serpentine, garnierite, pyrophyllite, talc, mica group, chlorite group, vermiculite, gyrolite, okenite, prehnite, apophyllite group, and chrysocolla; and tectosilicate minerals such as feldspar group, feldspathoid group, scapolite, zeolite group, leucite, ammonioleucite, inesite, danburite, helvite, and danalite are preferable, and kaolinite, mica group, and zeolite group are more preferable.

The "colloidal silica" that can be used in the present invention is a colloid of silicon dioxide (SiO₂) or a hydrate thereof.

The "alumina hydrate" includes aluminum hydroxide and is a generic term for crystalline substances and gels that can be represented by a chemical formula of Al₂O₃·nH₂O.

The particle diameter of the adsorptive inorganic particles used in the present invention in a water dispersion liquid varies depending on measurement conditions and the like, and is generally determined by particle diameter distribution measurement using water as a dispersion medium, and any particle diameter can be used as long as the effects of the present invention are not impaired. The median diameter of the adsorptive inorganic particles is preferably 10 nm or more, more preferably 20 nm or more, and preferably 10,000 nm or less, more preferably 5,000 nm or less from the viewpoint of efficiently and uniformly dispersing the adsorptive inorganic particles in a cell suspension.

In the measurement of the median diameter of the adsorptive inorganic particles, when the dispersion particle diameter is nanoscale, measurement by a photon correlation method is preferable, and the median diameter in a particle diameter distribution obtained as a diffusion coefficient equivalent diameter can be used for indication. The measurement can be performed by using a product obtained by dispersing the adsorptive inorganic particles in water and then diluting the dispersion liquid to about 0.1 mass%. The measuring instrument may be a commercially available instrument based on a photon correlation method. Examples thereof include SZ-100 series manufactured by HORIBA, Ltd. Other examples include Zetasizer Nano series manufactured by Malvern Instruments Ltd. and DLS-6500 series manufactured by OTSUKA ELECTRONICS CO., LTD.

When the adsorptive inorganic particles are microscale, the median diameter can be measured using a laser diffraction/scattering method. Examples of the measuring instrument include LA-960 series manufactured by HORIBA, Ltd., Mastersizer series manufactured by Malvern Instruments Ltd., and ELSZ series manufactured by OTSUKA ELECTRONICS CO., LTD.

The viscosity of the adsorptive inorganic particles used in the present invention can be any viscosity as long as the effects of the present invention are not impaired. For example, for the swellable layered silicate that can be preferably used in the present invention, the viscosity of a 1 mass% water dispersion liquid at 25°C measured under the condition of 60 rotations/min using a B-type viscometer is preferably 100 mPa · s or less, and more preferably 50 mPa · s or less. By setting the viscosity in such a range, accurate weighing of the dispersion liquid can be facilitated when the dispersion liquid is diluted in concentration adjustment for cell culture.

The adsorptive inorganic particles used in the present invention can adsorb proteins. The adsorption amount of proteins is performed using BSA as an exemplary reagent. For example, 0.2 mL of a 1 mass% dispersion liquid of adsorptive inorganic particles or 0.2 mL of distilled water for comparison is added to 1 mL of a 1 mg/mL BSA solution, the mixture is mixed by vortex and reacted with a rotator for 1 hour, then particles are precipitated using a centrifuge, and supernatant is collected to quantify BSA contained in the supernatant. The adsorption amount of BSA to the adsorptive inorganic particles can be quantified by comparison with the supernatant without addition of adsorptive inorganic particles.

In the quantification of proteins, Bradford method, BCA method, Lowly method and the like can be used. The adsorption amount of BSA per 1 mg of the adsorptive inorganic particles used in the present invention is 10 µg or more, more preferably 15 µg or more, more preferably 20 µg or more, more preferably 30 µg or more, more preferably 40 µg or more, more preferably 100 µg or more, more preferably 200 µg or more, and still more preferably 300 µg or more. The upper limit of the adsorption amount of BSA per 1 mg of the adsorptive inorganic particles is 500 µg or less, and preferably 490 µg or less. It is expected that by adsorption of proteins, inorganic particles act as a scaffold capable of efficiently providing a growth factor to cells.

The adsorptive inorganic particles used in the present invention may be natural products or may be synthesized according to a conventional method.

When natural products are used as the adsorptive inorganic particles, though the natural products may contain contaminants and impurities, from the viewpoint of promoting the formation of spheroids, contaminants and impurities are preferably removed.

Examples of the synthesis method of the swellable layered silicate include a hydrothermal synthetic process, a fusion synthetic process, a highpressure synthetic process, a solid-state reaction process, a flame fusion process, and a transformation process. As a synthesis method of the swellable layered silicate, a method described in JP-A-2008-13401 can be referred to. As a main synthesis method of colloidal silica, a gas phase synthesis method such as aerogyl synthesis by thermal decomposition of silicon tetrachloride, a method in which water glass is used as a raw material, and a liquid phase synthesis method such as hydrolysis of alkoxide are exemplary. Alumina hydrates are generally produced by the Bayer process, in which bauxite is dissolved in sodium hydroxide at high temperature.

In the present invention, commercially available adsorptive inorganic particles can also be used.

Examples of commercially available products of the swellable layered silicate include Kunipier-F (median diameter: 347.4 nm, BSA adsorption amount: 222 µg/mg, cation exchange capacity: 108 meq/100 g, viscosity of 1 mass% water dispersion liquid at 25°C: 4 mPa · s), smectone-SA (median diameter: 85.7 nm, BSA adsorption amount: 437 µg/mg, cation exchange capacity: 70 meq/100 g, viscosity of 1 mass% water dispersion liquid at 25°C: 5 mPa · s), smecton-SWN (median diameter: 64.4 nm, BSA adsorption amount: 485 µg/mg, cation exchange capacity: 49 meq/100 g, viscosity of 1 mass% water dispersion liquid at 25°C: 6 mPa · s), smectone-SWF (median diameter: 69.8 nm, BSA adsorption amount: 388 µg/mg, cation exchange capacity: 73 meq/100 g, viscosity of 1 mass% water dispersion liquid at 25°C: 16 mPa · s), smectone-ST (median diameter: 42.4 nm, BSA adsorption amount: 474 µg/mg, cation exchange capacity: 30 meq/100 g, viscosity of 1 mass% water dispersion liquid at 25°C: 2 mPa · s) (trade names, manufactured by KUNIMINE INDUSTRIES CO., LTD.), Somasif ME, Somasif MEB-3 (both are trade names, manufactured by Katakura & Co-op Agri Corporation), and PDM-5B, PDM-800 (both are trade names, manufactured by TOPY INDUSTRIES LIMITED.).

Examples of commercially available products of layered silicates other than the swellable layered silicate include Kaolin JP-100 (median diameter: 5.4 µm, BSA adsorption amount: 23 µg/mg, manufactured by Takehara Chemical), MK-100 (median diameter: 5.1 µm, BSA adsorption amount: 24 µg/mg, manufactured by Katakura & Co-op Agri Corporation), 1000 F, FG-15 F (both are trade names, manufactured by Nippon Talc Co., Ltd.), Hydrite TS90, Hydrite UF90 (both are trade names, manufactured by HAYASHI-KASEI CO., LTD.), Zeoal 4A (median diameter: 51.6 nm, BSA adsorption amount: 15 µg/mg, manufactured by Nakamura Choukou Co., Ltd.), Zeoal ZSM-5 (median diameter: 168.0 nm, BSA adsorption amount: 17 µg/mg, manufactured by Nakamura Choukou Co., Ltd.), HSZ-940NHA (median diameter: 3.2 µm, BSA adsorption amount: 34 µg/mg, manufactured by TOSOH CORPORATION), HSZ-820NHA (median diameter: 6.2 µm, BSA adsorption amount: 30 µg/mg, manufactured by TOSOH CORPORATION), and HSZ-320NAA (median diameter: 8.2 µm, BSA adsorption amount: 12 µg/mg, manufactured by TOSOH CORPORATION).

Examples of commercially available products of colloidal silica include SNOWTEX-C (median diameter: 45.9 nm, BSA adsorption amount: 47 µg/mg), SNOWTEX-CXS (median diameter: 46.0 nm, BSA adsorption amount: 89 µg/mg), SNOWTEX-30 (median diameter: 16.0 nm, BSA adsorption amount: 79 µg/mg), SNOWTEX Na type (ST-XS, ST-S, ST-50-T, ST-30L, ST-YL, ST-ZL, MP-1040, MP-2040, MP-4540M, ST-UP, ST-PS-S, ST-PS-M), SNOWTEX O type (ST-OXS, ST-OS, ST-O, ST-O-40, ST-OL, ST-OYL, ST-OUP, ST-PS-SO, ST-PS-MO), SNOWTEX N type (ST-NXS, ST-NS, ST-N, ST-N-40), SNOWTEX C type (ST-CM), and SNOWTEX AK type (ST-AK, ST-AK-L, ST-AK-YL) (all are trade names, manufactured by Nissan Chemical Corporation).

Examples of commercially available products of the alumina hydrate include Alumina Sol AS-200 (median diameter: 204.0 nm, BSA adsorption amount: 453 µg/mg, manufactured by Nissan Chemical Corporation), Alumina Sol AS-520-A (median diameter: 76.8 nm, BSA adsorption amount: 267 µg/mg, manufactured by Nissan Chemical Corporation), and Alumina Sol-10 A, Alumina Sol-A2, Alumina Sol-10C, and Alumina Sol-F1000 (all are trade names, manufactured by Kawaken Fine Chemicals Co., Ltd.).

Specific examples of other adsorptive inorganic particles include carbonates such as ALCAMIZER, MAGCELER, DHT-4A, DHT-4A-2, DHT-4C, and KYOWAAD 500 (all are trade names, manufactured by Kyowa Chemical Industry Co., Ltd.); magnesium oxide such as KYOWA MAG, KYOWA MAG MF, PYROKISUMA 5301, PYROKISUMA 3320, MAGSARAT, MAGMIC, and high-purity magnesium oxide 500-04R (all are trade names, manufactured by Kyowa Chemical Industry Co., Ltd.); magnesium hydroxide such as KISUMA (trade name, manufactured by Kyowa Chemical Industry Co., Ltd.); magnesium carbonate such as magnesium carbonate TT (trade name, manufactured by Naikai Salt Industries Co., Ltd.); magnesium silicate such as KYOWAAD 600 (trade name, manufactured by Kyowa Chemical Industry Co., Ltd.); aluminum silicate such as KYOWAAD 700 (trade name, manufactured by Kyowa Chemical Industry Co., Ltd.); aluminum hydroxide such as KYOWAAD 200 (trade name, manufactured by Kyowa Chemical Industry Co., Ltd.); magnesium oxide-aluminum oxide solid solution such as KW-2000 (trade name, manufactured by Kyowa Chemical Industry Co., Ltd.); hydrotalcite such as STABIACE HT (trade name, manufactured by SAKAI CHEMICAL INDUSTRY CO., LTD.); and hydroxyapatite such as SHAp, nano-SHAp (both are trade names, manufactured by SofSera Corporation), and HAP (trade name, manufactured by MP Biomedicals).

Further, the particle diameter of commercially available adsorptive inorganic particles that can be used in the present invention may be adjusted by pulverization for the purpose of improving dispersibility. The particle diameter of commercially available adsorptive inorganic particles can be further reduced by appropriately using, for example, a jet mill for dry pulverization and the like, a bead mill for wet pulverization and the like, and a pressure and moisture type pulverizer.

In the spheroid formation method of the present invention, a dispersion liquid in which the adsorptive inorganic particles are dispersed at a predetermined concentration is first prepared. In order to promote the formation of spheroids, the adsorptive inorganic particles need to be uniformly present in the dispersion liquid, and a dispersion in which the adsorptive inorganic particles are dispersed in advance in an aqueous solution such as water or a buffer solution and a dispersion medium are preferably mixed. Thereby, aggregation and precipitation of the adsorptive inorganic particles in the dispersion liquid can be suppressed.

The adsorptive inorganic particles used in the present invention may be used singly or in combination of two or more kinds thereof.

In the present invention, the concentration of the adsorptive inorganic particles in the dispersion liquid can be appropriately set. The solid content concentration of the adsorptive inorganic particles in the dispersion liquid is 0.001 mass% or more, preferably 0.003 mass% or more, and more preferably 0.01 mass% or more. The upper limit of the solid content concentration is less than 1.000 mass%, preferably 0.600 mass% or less, more preferably 0.500 mass% or less, more preferably 0.250 mass% or less, and still more preferably 0.125 mass% or less.

When the solid content concentration of the adsorptive inorganic particles is too low, the amount of the adsorptive inorganic particles is insufficient to promote spheroid formation. In addition, the components in the medium are excessively diluted when a mixed liquid having a predetermined concentration is formed. On the other hand, when the solid content concentration of the adsorptive inorganic particles is too high, the amount of aggregates increases when the adsorptive inorganic particles are mixed with medium components, which is not suitable for use in three-dimensional cell culture.

The dispersion medium for preparing the dispersion liquid of the adsorptive inorganic particles is not particularly limited as long as it has no toxicity to the cells that form spheroids and does not impair the proliferation and function of the cells. Specific examples thereof include water, a buffer, and a culture medium for cells. Examples of the buffer include phosphate-buffered saline (PBS), HEPES buffer, and Hanks buffer. Examples of the culture medium include D-MEM, E-MEM, MEMα, RPMI, and Ham's F-12. The dispersion liquid of the adsorptive inorganic particles that can be used in the present invention may further contain various additives as long as the effects of the present invention are not impaired.

Then, the prepared dispersion liquid of the adsorptive inorganic particles and a cell suspension in which a cell sample for forming spheroids is suspended in a solvent are mixed.

The solvent for preparing the cell suspension is not particularly limited as long as it has no toxicity to the cells that form spheroids and does not impair the proliferation and function of the cells. Specific examples thereof include water, a buffer, and a culture medium for cells. Examples of the buffer include phosphate-buffered saline (PBS), HEPES buffer, and Hanks buffer. Examples of the culture medium include D-MEM, E-MEM, MEMα, RPMI, and Ham's F-12. The cell suspension used in the present invention may further contain various additives as long as the effects of the present invention are not impaired. Examples thereof include phenol red indicators, buffers represented by HEPES, EDTA, L-glutamine, antibiotics such as penicillin, and antifungal agents such as fanginol.

At least one of the dispersion liquid of the adsorptive inorganic particles and the cell suspension contains a serum component generally used for the formation of spheroids. Examples of the serum component that can be used in the present invention include fetal bovine serum (FBS), bovine serum, horse serum, and porcine serum. The serum contains growth factors mainly including proteins, and growth factors such as cytokines may be used in a so-called serum-free medium. At least one of the dispersion liquid of the adsorptive inorganic particles and the cell suspension may contain a human platelet lysate (hPL).

In the present invention, by mixing the cell suspension and the dispersion liquid of the adsorptive inorganic particles, the serum component adsorbed to the adsorptive inorganic particles is supplied to the surface of cells.

The amount of the adsorptive inorganic particles to be mixed with the cell suspension may be an amount sufficient to obtain the spheroid formation promoting effect, and can be any amount according to the type of cells, the number of constituent cells, the culture environment and the like in the mixture of the cell suspension and the dispersion liquid of the adsorptive inorganic particles.

The solid content concentration of the swellable layered silicate in the mixture of the cell suspension and the dispersion liquid of the swellable layered silicate is 0.0003 w/v% or more, more preferably 0.0005 w/v% or more, more preferably 0.0006 w/v% or more, more preferably 0.0010 w/v% or more, and more preferably 0.0027 w/v% or more. The upper limit of the solid content concentration of the swellable layered silicic acid is 0.1667 w/v% or less, more preferably 0.0833 w/v% or less, and still more preferably 0.0417 w/v% or less.

When a phyllosilicate mineral among the above-mentioned silicates other than the swellable layered silicic acid is used as the adsorptive inorganic particles, the solid content concentration of the phyllosilicate mineral in the mixture of the cell suspension and the dispersion liquid of the phyllosilicate mineral is preferably 0.001 w/v% or more, more preferably 0.005 w/v% or more, more preferably 0.01 w/v% or more, and more preferably 0.02 w/v% or more. The upper limit of the solid content concentration of the phyllosilicate mineral is preferably less than 0.333 w/v%, preferably 0.1667 w/v% or less, more preferably 0.0833 w/v% or less, and still more preferably 0.0417 w/v% or less. When a tectosilicate mineral is used, the solid content concentration of the tectosilicate mineral in the mixture of the cell suspension and the dispersion liquid of the tectosilicate mineral is preferably 0.0005 w/v% or more, more preferably 0.001 w/v% or more, more preferably 0.002 w/v% or more, and more preferably 0.0025 w/v% or more. The upper limit of the solid content concentration of the tectosilicate mineral is preferably less than 0.333 w/v%, preferably 0.1667 w/v% or less, more preferably 0.08 w/v% or less, and still more preferably 0.04 w/v% or less.

When colloidal silica is used as the adsorptive inorganic particles, the solid content concentration of colloidal silica in the mixture of the cell suspension and the dispersion liquid of colloidal silica is preferably 0.0005 w/v% or more, more preferably 0.001 w/v% or more, more preferably 0.002 w/v% or more, and more preferably 0.0025 w/v% or more. The upper limit of the solid content concentration of the colloidal silica is preferably less than 0.04 w/v%, preferably 0.02 w/v% or less, more preferably 0.01 w/v% or less, and still more preferably 0.005 w/v% or less.

When alumina hydrate is used as the adsorptive inorganic particles, the solid content concentration of the alumina hydrate in the mixture of the cell suspension and the dispersion liquid of alumina hydrate is preferably 0.001 w/v% or more, more preferably 0.005 w/v% or more, more preferably 0.01 w/v% or more, more preferably 0.02 w/v% or more, and more preferably 0.04 w/v% or more. The upper limit of the solid content concentration of the alumina hydrate is preferably less than 0.5 w/v%, preferably 0.333 w/v% or less, more preferably 0.1667 w/v% or less, and still more preferably 0.08 w/v% or less.

When the solid content concentration of the adsorptive inorganic particles is too low, the amount of the adsorptive inorganic particles is insufficient to promote spheroid formation. On the other hand, when the solid content concentration of the adsorptive inorganic particles is too high, the adsorptive inorganic particles form a gel in the mixture, and the formation of spheroids is inhibited.

In the present invention, the solid content concentration of the adsorptive inorganic particles contained in the dispersion liquid of the adsorptive inorganic particles or the mixture obtained by mixing the dispersion liquid and the cell suspension can be determined according to a conventional method. For example, the solid content concentration of the composite adsorptive inorganic particles can be determined by drying a dispersion liquid or mixture containing the adsorptive inorganic particles such as the swellable layered silicate by freeze drying or the like, and measuring chemical composition analysis, the methylene blue adsorption amount, and the cation exchange capacity.

The cells contained in a cell sample used in the present invention may be any cells capable of forming a spheroid, and are preferably adherent cells. The cells contained in a cell suspension may be only one type or may include two or more types of cells.

The number of cells in the mixed liquid of the dispersion liquid of the adsorptive inorganic particles and the cell suspension is not particularly limited, and can be appropriately set in consideration of the cell type, culture conditions, the purpose of use of spheroids and the like. For example, the number is preferably 1 cells/µL or more and 10,000 cells/µL or less, and more preferably 10 cells/uL or more and 1000 cells/µL or less.

The cell used for the formation of spheroids is not particularly limited, and may be any cell such as a cell collected from an animal, a cell obtained by culturing a cell collected from an animal, a cell obtained by subjecting a cell collected from an animal to various treatments, and a cultured cell line. The kind of the animal from which the cell is derived is not particularly limited, and for example, a cell of mammalian animals such as humans, monkeys, dogs, cats, rabbits, pigs, cows, mice, and rats is preferably used.

When the cell to be used is a cell collected from an animal, the cell that can be used in the present invention may be any of epithelial tissue cells, muscle tissue cells, connective tissue cells, and neural tissue cells. The collection site is not particularly limited, and the cell may be any cell such as a somatic cell derived from bones, muscles, viscera, nerves, brains, bones, skins, and blood, a germ cell, and an embryonic stem cell (ES cell). Examples of the cell obtained by subjecting a cell to various treatments include an induced pluripotent stem cell (iPS cell) and a cell after induction of differentiation. The cultured cell line may be obtained from ATCC (American Type Culture Collection), ECACC (Europian Collection of Cell Cultures) or the like, or primary cultured cells of tissue cells collected from a clinical tissue can be used.

The cell that can be used in the present invention may be a cell in any cell cycle, may be an undifferentiated cell, or may be a cell after differentiation. The cell may be a normal cell or a cell collected from a pathological tissue such as a cancer tissue.

The cells contained in the mixture obtained by mixing the cell suspension and the dispersion liquid of the adsorptive inorganic particles are cultured in a vessel for spheroid formation culture according to an ordinarily method.

The vessel for culture that can be used in the present invention may be in the form of a vessel used for cell culture in a general spheroid culture method such as a culture plate for adherent cells and a culture plate for suspension cells, and the material, shape, and size are not particularly limited. A vessel in which adhesion other than adhesion between cells is suppressed, for example, a culture plate for suspension cells is more preferably used from the viewpoint of achieving the reduction of the amount of the adsorptive inorganic particles used. Examples of the material of the vessel for spheroid formation culture include, but are not limited to, glass, stainless, and plastic. Examples of the vessel for spheroid formation culture include, but are not limited to, dishes, tubes, flasks, bottles, and plates.

The treatment and operation for realizing a scaffold may be performed in the vessel for spheroid formation culture. However, as described above, adsorptive inorganic particles such as a swellable layered silicate which also exhibit a function as a scaffold material in a dispersion liquid are preferable because culture can be performed without using a generally used scaffold material from the viewpoint of suppressing cost.

The culture conditions in the vessel for spheroid formation culture may be an environment suitable for the cell type to be used, and for example, a commercially available culture medium recommended for the culture of the cell type to be used can be used, and the temperature conditions or the like recommended for the culture of the cell type to be used can be used. The culture time can also be any time according to the cell type to be used, the number of cells, and the size of the target spheroid.

For example, a mixture of the dispersion liquid of the adsorptive inorganic particles such as a swellable layered silicate and the cell suspension is held under an environment suitable for cell culture, for example, in a 5% CO₂ atmosphere under an environment of 37°C for any time. This can quickly achieve spheroid formation.

The kit for spheroid formation includes a spheroid formation promoter including adsorptive inorganic particles as an active ingredient. The kit for spheroid formation may contain a vessel for spheroid formation culture, a dispersion medium for preparing a dispersion liquid of adsorptive inorganic particles such as a swellable layered silicate, a solvent for preparing a cell suspension, a serum component necessary for the formation of spheroids, a cell culture medium and the like. Thus, spheroid formation can be performed more simply and in a short time by making a kit of reagents, instruments and the like necessary for promoting spheroid formation.

### EXAMPLES

Hereinafter, the present invention will be described in more detail based on Examples, but the present invention is not limited to the following Examples.

In the following Examples, when the median diameter of various adsorptive inorganic particles was nanoscale, the median diameter was measured using a 0.1 mass% water dispersion liquid of inorganic particles and a nanoparticle analyzer (nano Partica SZ-100 V2, manufactured by HORIBA, Ltd.). For microscale particles, the median diameter was measured using a laser diffraction/scattering type particle diameter distribution measuring instrument (Partica LA -950 V2, manufactured by HORIBA, Ltd.).

The BSA adsorption amount to the inorganic particles was quantified by adding 0.2 mL of a dispersion liquid of 1 mass% of adsorptive inorganic particles or distilled water as a blank to 1 mL of a 1 mg/mL BSA solution, stirring the mixture by vortex, then reacting the mixture with a rotator (MTR-103, manufactured by AS ONE Corporation) for 1 hour, then precipitating the particles using a centrifuge to collect a supernatant, quantifying BSA by Bradford method, and comparing the amount with that of the blank. A 1 mg/mL BSA solution was prepared by dissolving BSA (manufactured by FUJIFILM Wako Pure Chemical Corporation) powder in distilled water. Centrifugation was performed at 15000 G for 15 minutes using a high-speed centrifuge (CT15E, manufactured by Hitachi Koki Holdings Co., Ltd.). BSA in the supernatant was quantified by measuring the absorbance at 595 nm with a spectrophotometer (ASV11D-H, manufactured by AS ONE Corporation) using TaKaRa Bradford Protein Assay Kit (manufactured by Takara Bio Inc.). A value obtained by subtracting the amount of BSA in the inorganic particle dispersion liquid reaction supernatant from the amount of BSA in the blank was defined as the BSA adsorption amount to the adsorptive inorganic particles.

### (Test Example 1) Three-dimensional cell culture using swellable layered silicate [Preparation Example 1-1]

Fluorinated sodium hectorite (trade name: smectone-SWF, manufactured by KUNIMINE INDUSTRIES CO., LTD., hereinafter, also simply referred to as "SWF") (100 g) was put into an 80 mass% aqueous isopropyl alcohol solution (water/isopropyl alcohol = 20/80 (mass ratio)) (2 kg). Thereafter, the mixture was stirred at 25°C for 20 minutes at 100 rpm using a propeller stirrer (manufactured by AS ONE Corporation). The obtained suspension was filtered by suction with Nutsche, and subjected to solid-liquid separation to obtain a dehydrated cake. The obtained cake was washed by passing 2 kg of an 80 mass% aqueous isopropyl alcohol solution (water/isopropyl alcohol = 20/80 (mass ratio)) through the cake for filtration, and the same washing by liquid passing for filtration were repeated twice. The dehydrated cake after washing was collected and dried in a dryer at 105°C to collect a dried powder.

The cation exchange capacity of the obtained powder was measured and found to be 73.3 meq/100 g. Thereafter, the powder and distilled water were mixed using a rotation-revolution mixer (manufactured by THINKY CORPORATION) to obtain a dispersion liquid 1 having a solid content concentration of hectorite of 1 mass%. The median diameter of the fluorinated sodium hectorite in the dispersion liquid was 42.1 nm. Further, the BSA adsorption amount to 1 mg of fluorinated sodium hectorite was 388 µg/mg.

### [Preparation Example 1-2]

A dispersion liquid 2 having a solid content concentration of hectorite of 1 mass% was obtained in the same manner as in Preparation Example 1 except that a sodium hectorite powder (trade name: smectone-SWN, manufactured by KUNIMINE INDUSTRIES CO., LTD., hereinafter, also simply referred to as "SWN") was used instead of the fluorinated sodium hectorite powder.

The cation exchange capacity of the obtained powder was 53.1 meq/100 g. The median diameter of sodium hectorite in the dispersion liquid was 71.0 nm. Further, the BSA adsorption amount to 1 mg of sodium hectorite was 485 µg/mg.

### [Three-dimensional cell culture]

### (1) Preparation of medium-1

To 100 parts by mass of RPMI-1640 (manufactured by FUJIFILM Wako Pure Chemical Corporation, hereinafter also simply referred to as "RPMI"), D-MEM (manufactured by FUJIFILM Wako Pure Chemical Corporation, hereinafter also simply referred to as "DMEM"), or E-MEM (manufactured by FUJIFILM Wako Pure Chemical Corporation, hereinafter also simply referred to as "EMEM") as a culture medium for cell culture, 10 parts by mass of FBS (manufactured by Gibco) was added. A cell suspension in which the number of cells was adjusted using various cells using the medium was prepared, and 100 µL of the obtained suspension was seeded on two types of 96 well plates (a culture plate for adherent cells) (trade name: Tissue culture plate VTC-P96, manufactured by Violamo, hereinafter also simply referred to as "Violamo") and a culture plate for suspension cells (trade name: Non-treated 96 well microplate, manufactured by Iwaki, hereinafter also simply referred to as "Iwaki")). The number of cells seeded on the plates is shown in Tables 1 to 4. The cells used were a human pancreatic cancer cell PANC-1 strain (hereinafter, also simply referred to as "PANC-1"), a human kidney cancer cell ACHN strain (hereinafter, also simply referred to as "ACHN"), a human glioma (astrocytoma) cell U-251MG strain (hereinafter, also simply referred to as "U251MG"), and a human bladder cancer cell UMUC3 (hereinafter, also simply referred to as "UMUC3"), and these cells were obtained from the American Type Culture Collection (ATCC).

### (2) Preparation of medium-2

FBS (manufactured by Gibco) (5 parts by mass) was mixed with D-MEM (manufactured by FUJIFILM Wako Pure Chemical Corporation) (100 parts by mass) as a medium for cell culture. A cell suspension in which the number of cells was adjusted using various cells using the medium was prepared, and 100 µL of the obtained suspension was seeded on a 96 well plate (culture plate for suspension cells) (trade name: Non-treated 96 well microplate, manufactured by Iwaki). The number of cells seeded on the plate is shown in Table 4. The cells used were a human glioma cell KNS-81 strain (hereinafter, also simply referred to as "KNS81") and a human glioblastoma cell LN-299 strain (hereinafter, also simply referred to as the "LN299"), and these cells were obtained from the American Type Culture Collection (ATCC).

### (3) Addition of swellable layered silicate

Using sterile water, dispersion liquids obtained by diluting the dispersions 1 and 2 prepared in Preparation Examples 1-1 and 1-2 2 times, 4 times, 8 times, 16 times, 32 times, 64 times, 128 times, 256 times, or 512 times were prepared. Dispersion liquids 1 and 2 and dispersion liquids prepared by diluting them (each corresponding to 1, 0.5, 0.25, 0.125, 0.0625, 0.031, 0.016, 0.008, 0.004, 0.002 w/v%) (50 µL) were added to the cell suspension (100 µL) seeded on a 96 well plate. The concentrations of the swellable layered silicate in the media at the time of final culture were 0.33, 0.17, 0.083, 0.042, 0.021, 0.010, 0.0052, 0.0026, 0.0013, and 0.00065 w/v%. The plate after the addition of the swellable layered silicate was cultured in an incubator at 37°C in a 5% CO₂ atmosphere.

### (4) Evaluation of spheroid formation

The plate after culture was observed with a microscope to confirm the presence or absence of formation of spheroids.

The results of the presence or absence of formation of spheroids are shown in Tables 1 to 4. The results of three-dimensional cell culture performed without using the dispersion liquids prepared in Preparation Examples 1-1 and 1-2 are also shown. Regarding the "State of cells after culture" in Tables 1 to 4, "One-dimensional" means that the observed cell was a cell that existed alone in a spherical form. The term "Two-dimensional" means that the observed cells were cells present in one layer that were amorphous and adhered to the plate, and the formation of spheroids was not confirmed. The term "Three-dimensional" means that formation of spheroids was confirmed. The term "Three-dimensional like" means that spheroid like cells, which were not completely spherical but partially spherical, were present.

Photomicrographs showing the state of cells after culturing a human pancreatic cancer cell PANC-1 strain or a human kidney cancer cell ACHN strain for 3 days in a mixture in which a swellable layered silicate was dispersed so that the solid content concentration of the swellable layered silicate would be 0.0052 w/v% using a culture plate for suspension cells are shown in Fig. 1 (Example 8), Fig. 2 (Example 20), Fig. 3 (Example 12), and Fig. 4 (Example 24). Further, photomicrographs showing the state of cells after culturing a human pancreatic cancer cell PANC-1 strain or a human kidney cancer cell ACHN strain for 3 days in a cell suspension not containing a swellable layered silicate are shown in Fig. 5 (Reference Example 2) and Fig. 6 (Reference Example 4).

Further, photomicrographs showing changes in the state of cells 1 day, 3 days, and 7 days after culturing a human pancreatic cancer cell PANC-1 strain and a human kidney cancer cell ACHN strain in a mixture in which a swellable layered silicate was dispersed so that the solid content concentration of the swellable layered silicate of the dispersion liquid 2 would be 0.0052 w/v% are shown in Figs. 7 and 8, respectively.

**Table 1**

| | REx. 1 | Ex. 1 | Ex. 2 | Ex. 3 | Ex. 4 | Ex. 5 |
|---|---|---|---|---|---|---|
| Cell type | PANC-1 | PANC-1 | PANC-1 | PANC-1 | PANC-1 | PANC-1 |
| Cells seeded | 1×10³ | 5×10³ | 5×10³ | 5×10³ | 5×10³ | 5×10³ |
| Plate | Violamo | Violamo | Violamo | Violamo | Violamo | Violamo |
| Medium | RPMI | RPMI | RPMI | RPMI | RPMI | RPMI |
| Spheroid formation promoter | None | SWF | SWF | SWF | SWF | SWF |
| Solid content concentration (w/v%) of swellable layered silicate in dispersion liquid | - | 0.0078 | 0.016 | 0.031 | 0.063 | 0.13 |
| Solid content concentration (w/v%) of swellable layered silicate in mixture | - | 0.0026 | 0.0052 | 0.010 | 0.021 | 0.042 |
| State of cell after culture | Two-dimensional | Three-dimensional | Three-dimensional | Three-dimensional | Three-dimensional | Three-dimensional |

| | Ex. 6 | Ex. 7 | CEx. 1 | REx. 2 | Ex. 8 |
|---|---|---|---|---|---|
| Cell type | PANC-1 | PANC-1 | PANC-1 | PANC-1 | PANC-1 |
| Cells seeded | 5×10³ | 5×10³ | 1×10⁴ | 1×10³ | 1×10³ |
| Plate | Violamo | Violamo | Violamo | IWAKI | IWAKI |
| Medium | RPMI | RPMI | RPMI | RPMI | RPMI |
| Spheroid formation promoter | SWF | SWF | SWF | None | SWF |
| Solid content concentration (w/v%) of swellable layered silicate in dispersion liquid | 0.13 | 0.25 | 1 | - | 0.016 |
| Solid content concentration (w/v%) of swellable layered silicate in mixture | 0.042 | 0.083 | 0.333 | - | 0.0052 |
| State of cell after culture | Three-dimensional | Three-dimensional | Two-dimensional | One-dimensional | Three-dimensional |

| | | | | | |
|---|---|---|---|---|---|
| Remarks: 'REx.' means Reference Example according to this invention, 'CEx.' means Comparative Example, and 'Ex.' means Example according to this invention. | | | | | |

**Table 2**

| | REx. 3 | Ex. 9 | Ex. 10 | Ex. 11 | CEx. 2 |
|---|---|---|---|---|---|
| Cell type | ACHN | ACHN | ACHN | ACHN | ACHN |
| Cells seeded | 1×10³ | 5×10³ | 5×10³ | 5×10³ | 1×10⁴ |
| Plate | Violamo | Violamo | Violamo | Violamo | Violamo |
| Medium | RPMI | RPMI | RPMI | RPMI | RPMI |
| Spheroid formation promoter | None | SWF | SWF | SWF | SWF |
| Solid content concentration (w/v%) of swellable layered silicate in dispersion liquid | - | 0.0078 | 0.063 | 0.25 | 1 |
| Solid content concentration (w/v%) of swellable layered silicate in mixture | - | 0.0026 | 0.021 | 0.083 | 0.333 |
| State of cell after culture | Two-dimensional | Three-dimensional | Three-dimensional | Three-dimensional | Three-dimensional like |

| | REx. 4 | Ex. 12 | Ex. 13 | Ex. 14 | Ex. 15 | Ex. 16 |
|---|---|---|---|---|---|---|
| Cell type | ACHN | ACHN | ACHN | ACHN | ACHN | ACHN |
| Cells seeded | 1×10³ | 1×10³ | 1×10³ | 1×10³ | 1×10³ | 1×10³ |
| Plate | IWAKI | IWAKI | IWAKI | IWAKI | IWAKI | IWAKI |
| Medium | RPMI | RPMI | RPMI | RPMI | RPMI | RPMI |
| Spheroid formation promoter | None | SWF | SWF | SWF | SWF | SWF |
| Solid content concentration (w/v%) of swellable layered silicate in dispersion liquid | - | 0.016 | 0.031 | 0.063 | 0.13 | 0.25 |
| Solid content concentration (w/v%) of swellable layered silicate in mixture | - | 0.0052 | 0.010 | 0.021 | 0.042 | 0.083 |
| State of cell after culture | Two-dimensional | Three-dimensional | Three-dimensional | Three-dimensional | Three-dimensional | Three-dimensional |

| | | | | | | |
|---|---|---|---|---|---|---|
| Remarks: 'REx.' means Reference Example according to this invention, 'CEx.' means Comparative Example, and 'Ex.' means Example according to this invention. | | | | | | |

**Table 3**

| | Ex. 17 | Ex. 18 | Ex. 19 | Ex. 20 | Ex. 21 | Ex. 22 |
|---|---|---|---|---|---|---|
| Cell type | PANC-1 | PANC-1 | PANC-1 | PANC-1 | ACHN | ACHN |
| Cells seeded | 5×10³ | 5×10³ | 5×10³ | 1×10³ | 5×10³ | 5×10³ |
| Plate | Violamo | Violamo | Violamo | IWAKI | Violamo | Violamo |
| Medium | RPMI | RPMI | RPMI | RPMI | RPMI | RPMI |
| Spheroid formation promoter | SWN | SWN | SWN | SWN | SWN | SWN |
| Solid content concentration (w/v%) of swellable layered silicate in dispersion liquid | 0.0078 | 0.063 | 0.25 | 0.016 | 0.0078 | 0.25 |
| Solid content concentration (w/v%) of swellable layered silicate in mixture | 0.0026 | 0.021 | 0.083 | 0.0052 | 0.0026 | 0.083 |
| State of cell after culture | Three-dimensional | Three-dimensional | Three-dimensional | Three-dimensional | Three-dimensional | Three-dimensional |

| | Ex. 23 | Ex. 24 | Ex. 25 | Ex. 26 | Ex. 27 | Ex. 28 |
|---|---|---|---|---|---|---|
| Cell type | ACHN | ACHN | ACHN | ACHN | ACHN | ACHN |
| Cells seeded | 2×10⁴ | 1×10³ | 1×10³ | 1×10³ | 1×10³ | 1×10³ |
| Plate | Violamo | IWAKI | IWAKI | IWAKI | IWAKI | IWAKI |
| Medium | RPMI | RPMI | RPMI | RPMI | RPMI | RPMI |
| Spheroid formation promoter | SWN | SWN | SWN | SWN | SWN | SWN |
| Solid content concentration (w/v%) of swellable layered silicate in dispersion liquid | 0.5 | 0.016 | 0.031 | 0.063 | 0.13 | 0.25 |
| Solid content concentration (w/v%) of swellable layered silicate in mixture | 0.1667 | 0.0052 | 0.010 | 0.021 | 0.042 | 0.083 |
| State of cell after culture | Three-dimensional | Three-dimensional | Three-dimensional | Three-dimensional | Three-dimensional | Three-dimensional |

| | | | | | | |
|---|---|---|---|---|---|---|
| Remarks: 'Ex.' means Example according to this invention. | | | | | | |

**Table 4**

| | Ex. 29 | Ex. 30 | Ex. 31 | Ex. 32 |
|---|---|---|---|---|
| Cell type | KNS81 | KNS81 | LN299 | LN299 |
| Cells seeded | 4×10³ | 4×10³ | 4×10³ | 4×10³ |
| Plate | IWAKI | IWAKI | IWAKI | IWAKI |
| Medium | DMEM | DMEM | DMEM | DMEM |
| Spheroid formation promoter | SWF | SWN | SWF | SWN |
| Solid content concentration (w/v%) of swellable layered silicate in dispersion liquid | 0.0039 | 0.0039 | 0.0039 | 0.0039 |
| Solid content concentration (w/v%) of swellable layered silicate in mixture | 0.0013 | 0.0013 | 0.0013 | 0.0013 |
| State of cell after culture | Three-dimensional | Three-dimensional | Three-dimensional | Three-dimensional |

| | Ex. 33 | Ex. 34 | Ex. 35 | Ex. 36 |
|---|---|---|---|---|
| Cell type | U251MG | U251MG | UMUC3 | UMUC3 |
| Cells seeded | 4×10³ | 4×10³ | 5×10⁴ | 5×10⁴ |
| Plate | IWAKI | IWAKI | Violamo | Violamo |
| Medium | EMEM | EMEM | DMEM | DMEM |
| Spheroid formation promoter | SWF | SWN | SWF | SWN |
| Solid content concentration (w/v%) of swellable layered silicate in dispersion liquid | 0.0078 | 0.0078 | 0.016 | 0.016 |
| Solid content concentration (w/v%) of swellable layered silicate in mixture | 0.0026 | 0.0026 | 0.0052 | 0.0052 |
| State of cell after culture | Three-dimensional | Three-dimensional | Three-dimensional | Three-dimensional |

| | | | | |
|---|---|---|---|---|
| Remarks: 'Ex.' means Example according to this invention. | | | | |

In the absence of the swellable layered silicate (Reference Examples 1 to 4), spheroids were not formed even when cell culture was performed for 3 days (see Figs. 5 and 6).

On the other hand, when three-dimensional cell culture was performed in the presence of the swellable layered silicate (Examples 1 to 36), spheroids were formed in about 3 days, 1 day at the shortest, after the start of culture (see Figs. 1 to 4 and 7 and 8).

These results indicate that the swellable layered silicate dispersed in a cell suspension under three-dimensional cell culture conditions has an action of promoting aggregation or organization of cells and promoting formation of spheroids irrespective of cell types. Further, by using the swellable layered silicate, spheroids can be produced without using a special scaffold material, culture vessel, or medium.

Further, the results shown in Tables 1 to 4 also indicate that to produce spheroids, the solid content concentration of the swellable layered silicate in the mixture needs to be set within a predetermined range.

Specifically, when culture was performed without addition of the swellable layered silicate to the medium, formation of spheroids was not confirmed, and all the cultured cells were monolayer cells (Reference Examples 1 and 3). On the other hand, when the solid content concentration of the swellable layered silicate in the mixture was too high, a pure spheroid was not formed (Comparative Example 1) or a non-uniform spheroid like state was observed (Comparative Example 2).

Against this, as shown in the results of Examples 1 to 36, to form spheroids, it is important to set the solid content concentration of the swellable layered silicate dispersed in the cell suspension within a predetermined range. Further, as also shown in Figs. 7 and 8, formation of spheroids was observed the day after the start of culture (see Figs. 7(a) and 8(a)), and the growth of spheroids was confirmed on Day 3 after the start of culture and further by Day 7 (see Figs. 7(b) and 7(c) and Figs. 8(b) and 8(c)).

### (Test Example 2) Three-dimensional cell culture using various adsorptive inorganic particles

### [Preparation Example 2-1]

Colloidal silica (trade name: SNOWTEX ST-C, manufactured by Nissan Chemical Corporation, solid content concentration: 20.5 mass%) and distilled water were mixed to obtain a 1 mass% colloidal silica dispersion liquid.

The median diameter of the colloidal silica in the dispersion liquid was 45.9 nm. Further, the BSA adsorption amount to 1 mg of the colloidal silica was 47 µg/mg.

### [Preparation Example 2-2]

Colloidal silica (trade name: SNOWTEX ST-CXS, manufactured by Nissan Chemical Corporation, solid content concentration: 15 mass%) and distilled water were mixed to obtain a 1 mass% colloidal silica dispersion liquid.

The median diameter of the colloidal silica in the dispersion liquid was 45.5 nm. Further, the BSA adsorption amount to 1 mg of the colloidal silica was 89 µg/mg.

### [Preparation Example 2-3]

Alumina hydrate particles (trade name: Alumina Sol AS-200, manufactured by Nissan Chemical Corporation, solid content concentration: 10.6 mass%) and distilled water were mixed to obtain a 1 mass% alumina hydrate dispersion liquid.

The median diameter of the alumina hydrate particles in the dispersion liquid was 204.0 nm. Further, the BSA adsorption amount to 1 mg of the alumina hydrate particles was 453 µg/mg.

### [Preparation Example 2-4]

Alumina hydrate particles (trade name: Alumina Sol AS-520-A, manufactured by Nissan Chemical Corporation, solid content concentration: 21 mass%) and distilled water were mixed to obtain a 1 mass% alumina hydrate dispersion liquid.

The median diameter of the alumina hydrate particles in the dispersion liquid was 77.0 nm. Further, the BSA adsorption amount to 1 mg of the alumina hydrate particles was 267 µg/mg.

### [Preparation Example 2-5]

Phyllosilicate mineral particles (trade name: Kaolin JP-100, manufactured by Takehara Chemical) and distilled water were mixed to obtain a 1 mass% phyllosilicate mineral dispersion liquid.

The median diameter of the phyllosilicate mineral particles in the dispersion liquid was 5.4 µm. Further, the BSA adsorption amount to 1 mg of the phyllosilicate mineral particles was 23 µg/mgg.

### [Preparation Example 2-6]

Phyllosilicate mineral particles (mica) (trade name: MK-100, manufactured by Katakura & Co-op Agri Corporation) and distilled water were mixed to obtain a 1 mass% phyllosilicate mineral dispersion liquid.

The median diameter of the phyllosilicate mineral particles in the dispersion liquid was 5.1 µm. Further, the BSA adsorption amount to 1 mg of the phyllosilicate mineral particles was 24 µg/mg.

### [Three-dimensional cell culture]

To 100 parts by mass of RPMI (manufactured by FUJIFILM Wako Pure Chemical Corporation), 10 parts by mass of FBS (manufactured by Gibco) was added to prepare a medium. Using the obtained medium, the dispersion liquids obtained in Preparation Examples 2-1 to 2-6 were diluted and injected into a 96 well plate (Iwaki). Thereto was seeded a human colon cancer cell HT29 strain (hereinafter, also simply referred to as "HT29") at 1,000 cells/50 µL. The HT29 strain was obtained from the American Type Culture Collection (ATCC). The plate after seeding of cells was cultured in an incubator at 37°C in a 5% CO₂ atmosphere for 4 days.

The plate after culture was observed with a fluorescence microscope (trade name: Biozero, manufactured by KEYENCE CORPORATION) to confirm the presence or absence of formation of spheroids. The results are shown in Table 9.

As shown in Fig. 9, spheroids are also formed by using the dispersion liquids obtained in Preparation Examples 2-1 to 2-6.

Further, spheroid formation was performed at changed concentrations of various inorganic particles contained in the dispersion liquids obtained in Preparation Examples 2-1 to 2-6 to determine the solid content concentration of the inorganic particles in which spheroids are formed.

The results are shown in Table 5.

**Table 5**

| | Solid content concentration (w/v%) of inorganic particles in which spheroids are formed |
|---|---|
| Preparation Example 2-1 | 0.005 to 0.01 |
| Preparation Example 2-2 | 0.0025 to 0.005 |
| Preparation Example 2-3 | 0.02 to 0.08 |
| Preparation Example 2-4 | 0.02 to 0.08 |
| Preparation Example 2-5 | 0.02 to 0.04 |
| Preparation Example 2-6 | 0.02 to 0.08 |

The results shown in Table 5 indicate that to produce spheroids, the solid content concentration of various inorganic particles in the mixture needs to be set within a predetermined range.

### (Test Example 3) Three-dimensional cell culture using various adsorptive inorganic particles

### [Preparation Example 3-1]

Tectosilicate mineral particles (zeolite) (trade name: Zeoal-ZSM-5, manufactured by Nakamura Choukou Co., Ltd., solid content concentration: 32 mass%) and distilled water were mixed to obtain a 1 mass% tectosilicate dispersion liquid.

The median diameter of the tectosilicate mineral particles in the dispersion liquid was 248.5 nm. Further, the BSA adsorption amount to 1 mg of the tectosilicate mineral particles was 17 µg/mg.

### [Preparation Example 3-2]

Tectosilicate mineral particles (zeolite) (trade name: HSZ-940NHA, manufactured by TOSOH CORPORATION) and distilled water were mixed to obtain a 1 mass% tectosilicate dispersion liquid.

The median diameter of the tectosilicate mineral particles in the dispersion liquid was 3.2 µm. Further, the BSA adsorption amount to 1 mg of the tectosilicate mineral particles was 34 µg/mg.

### [Preparation Example 3-3]

Magnesium oxide particles (trade name: KYOWA MAG MF-150, manufactured by Kyowa Chemical Industry Co., Ltd.) and distilled water were mixed to obtain a 1 mass% magnesium oxide dispersion liquid.

The median diameter of the magnesium oxide particles in the dispersion liquid was 6.6 µm. Further, the BSA adsorption amount to 1 mg of the magnesium oxide particles was 104 µg/mg.

### [Preparation Example 3-4]

Hydroxyapatite particles (trade name: SHAp, manufactured by SofSera Corporation) and distilled water were mixed to obtain a 1 mass% hydroxyapatite dispersion liquid.

The median diameter of the hydroxyapatite particles in the dispersion liquid was 7.0 µm. Further, the BSA adsorption amount to 1 mg of the hydroxyapatite particles was 17 µg/mg.

### [Three-dimensional cell culture]

To 100 parts by mass of RPMI (manufactured by FUJIFILM Wako Pure Chemical Corporation), 10 parts by mass of FBS (manufactured by Gibco) was added to prepare a medium. Using the obtained medium, the dispersion liquids obtained in Preparation Examples 3-1 to 3-4 were diluted and injected into a 96 well plate (Iwaki). A human kidney cancer cell ACHN strain was seeded therein at 1,000 cells/50 µL. The plate after seeding of cells was cultured in an incubator at 37°C in a 5% CO₂ atmosphere for 5 days.

The plate after culture was observed with a fluorescence microscope (trade name: Biozero, manufactured by KEYENCE CORPORATION) to confirm the presence or absence of formation of spheroids. The results are shown in Table 10.

As shown in Fig. 10, spheroids are also formed by using the dispersion liquids obtained in Preparation Examples 3-1 to 3-4.

### (Test Example 4) Confirmation of adsorbability of inorganic particles

For the purpose of clarifying the effect and location of the adsorptive inorganic particles in the spheroid formed by the present invention, a fluorescence marker was retained in SWF, and the state of the spheroid was observed.

As a fluorescent marker for SWF, rhodamine 6G (manufactured by KANTO CHEMICAL CO., INC.) was dissolved in distilled water to prepare a 2 mM aqueous solution. To 1000 µL of the dispersion liquid of Preparation Example 1-1, 500 µL of distilled water and 500 µL of a 2 mM aqueous rhodamine 6G solution were added and the mixture was mixed with a vortex mixer. The dispersion liquid after mixing was passed through a syringe filter (pore size: 0.45 µm) to obtain a dispersion liquid (0.5 wt% R6G-SWF dispersion liquid) of composite particles (R6G-SWF) in which rhodamine 6G was retained in SWF.

FBS (manufactured by Gibco) (10 parts by mass) was mixed with DMEM (manufactured by FUJIFILM Wako Pure Chemical Corporation) (100 parts by mass) to obtain a 10% FBS/D-MEM medium. The medium was used to dilute the 0.5 wt% R6G-SWF dispersion liquid, and the concentration of the composite particles was adjusted to 0.02 w/v%. Further, the medium was passed through a syringe filter (pore size: 5 µm), and then the medium was added to a 96 well plate (manufactured by Iwaki) at 50 µL/well. A human colon cancer cell SW620 strain (1000 cells/50 µL) adjusted with 10% FBS/D-MEM medium was seeded to this at 50 µL/well. The SW620 strain was obtained from the American Type Culture Collection (ATCC). The plate after seeding of cells was cultured in an incubator at 37°C in a 5% CO₂ atmosphere.

On Day 5 from the start of culture, nuclei were stained using NucBlue Live ReadyProbes Reagent (manufactured by ThermoFisher Scientific), spheroids formed in the 96 well plate (manufactured by Greiner Bio-One) were transferred, and Z-stack imaging was performed at 0.3 µm intervals using a confocal laser microscope (CellVoyager CV8000, manufactured by Yokogawa Electric Corporation, excitation wavelength: 405 nm · 561 nm, detection wavelength: 445/45 nm · 600/37 nm, 60 times). Based on the obtained data, an MIP (Maximum Intensity Projection) image was created with an analysis software (CellPathfinder, manufactured by Yokogawa Electric Corporation).

The results are shown in Fig. 11.

In the obtained MIP image, nuclei of living cells are stained blue. In the MIP image, the location of cells forming spheroids is clear. According to the present invention, living cells are aggregated to form spheroids, and R6G-SWF stained in red is distributed around living cells. As described above, inorganic particles are adsorbed on the cell surface to promote the formation of spheroids.

R6G-SWF is abundantly present in the central part of spheroids, which shows that R6G-SWF is present in the cell surface layer at the early stage of the formation of spheroids, and the spheroid itself proliferates and grows outward.

From the above results, adsorptive inorganic particles can be used as an active ingredient of a spheroid formation promoter. Further, in three-dimensional cell culture, by setting the solid content concentration of the adsorptive inorganic particles dispersed in a cell suspension within a predetermined range, aggregation or organization of cells is promoted irrespective of the cell type, and cells in a spheroid form can be easily and quickly cultured.

The present application claims priority of Patent Application No. 2020-049062, filed in Japan on March 19, 2020.

## Claims

1. A method of producing animal cells in a spheroid form, comprising the steps of:
mixing a dispersion liquid of inorganic particles having adsorbability and a cell suspension to obtain a mixture in which the inorganic particles having adsorbability are dispersed, and
culturing a cell contained in the obtained mixture to form a spheroid,
wherein the solid content concentration of the adsorptive inorganic particles in the dispersion liquid is 0.001 mass% or more and less than 1.000 mass%,
wherein the inorganic particles act as spheroid formation promoter, and
wherein the inorganic particles are particles composed of at least a swellable layered silicate, wherein the solid content concentration of the swellable layered silicate in the mixture of the cell suspension and the dispersion liquid of the swellable layered silicate is 0.0003 w/v% or more and 0.1667 w/v% or less,
wherein an adsorption amount of bovine serum albumin per 1 mg of the inorganic particles having adsorbability is 10 µg or more and 500 µg or less.

2. The method according to claim 1,
wherein the inorganic particles are particles composed of at least one kind of swellable layered silicate selected from the group consisting of montmorillonite, hectorite, stevensite, saponite, beidellite, nontrite, sauconite, and swellable mica.

3. The method according to claim 1 or 2,
wherein the animal cell is a cell collected from an animal, a cell obtained by culturing a cell collected from an animal, a cell obtained by subjecting a cell collected from an animal to various treatments, or a cultured cell line, and/or wherein the cell is a mammalian animal cell.

4. The method according to any one of claims 1 to 3, wherein the animal cell is cultured in a culture plate for suspension cells to form a spheroid.

## Patentansprüche

1. Verfahren zur Herstellung von tierischen Zellen in Sphäroidform, umfassend die folgenden Schritte:
Mischen einer Dispersionsflüssigkeit aus anorganischen Partikeln mit Adsorbierbarkeit und einer Zellsuspension, um ein Gemisch zu erhalten, in dem die anorganischen Partikel mit Adsorbierbarkeit dispergiert sind, und
Kultivieren einer in der erhaltenen Mischung enthaltenen Zelle, um ein Sphäroid zu bilden,
wobei die Feststoffkonzentration der adsorbierbaren anorganischen Partikel in der Dispersionsflüssigkeit 0,001 Massenprozent oder mehr und weniger als 1,000 Massenprozent beträgt,
wobei die anorganischen Partikel die Sphäroidbildung fördern, und
wobei die anorganischen Partikel Partikel sind, die aus mindestens einem quellbaren Schichtsilikat zusammengesetzt sind, wobei die Feststoffkonzentration des quellbaren Schichtsilikats in der Mischung aus der Zellsuspension und der Dispersionsflüssigkeit des quellbaren Schichtsilikats 0,0003 Gew.Nolumen-% oder mehr und 0,1667 Gew.Nolumen-% oder weniger beträgt,
wobei die Adsorptionsmenge an Rinderserumalbumin pro 1 mg der anorganischen Partikel mit Adsorbierbarkeit 10 µg oder mehr und 500 µg oder weniger beträgt.

2. Verfahren nach Anspruch 1,
wobei die anorganischen Partikel Partikel sind, die aus mindestens einer Art quellfähigem Schichtsilikat zusammengesetzt sind, ausgewählt aus der Gruppe bestehend aus Montmorillonit, Hectorit, Stevensit, Saponit, Beidellit, Nontrit, Sauconit und quellfähigem Glimmer.

3. Verfahren nach Anspruch 1 oder 2,
wobei die tierische Zelle eine aus einem Tier gewonnene Zelle, eine Zelle, die dadurch erhalten wurde, dass eine aus einem Tier gewonnene Zelle kultiviert wurde, eine Zelle, die dadurch erhalten wurde, dass eine aus einem Tier gewonnene Zelle verschiedenen Behandlungen unterzogen wurde, oder eine kultivierte Zelllinie ist, und/oder
wobei die Zelle eine Säugetierzelle ist.

4. Verfahren nach einem der Ansprüche 1 bis 3, wobei die tierische Zelle in einer Kulturplatte für Suspensionszellen kultiviert wird, um ein Sphäroid zu bilden.

## Revendications

1. Procédé de production de cellules animales sous forme de sphéroïdes, comprenant les étapes suivantes:
mélanger un liquide de dispersion de particules inorganiques présentant une capacité d'adsorption et une suspension cellulaire pour obtenir un mélange dans lequel les particules inorganiques présentant une capacité d'adsorption sont dispersées, et
cultiver une cellule contenue dans le mélange obtenu pour former un sphéroïde,
dans lequel la concentration en matière solide des particules inorganiques adsorbantes dans le liquide de dispersion est de 0,001 % en masse ou plus et de 1,000 % en masse ou moins,
dans lequel les particules inorganiques agissent comme promoteur de formation de sphéroïdes, et
dans lequel les particules inorganiques sont des particules composées d'au moins un silicate stratifié gonflable, dans lequel la concentration en matière solide du silicate stratifié gonflable dans le mélange de la suspension cellulaire et du liquide de dispersion du silicate stratifié gonflable est de 0,0003 p/v % à 0,1667 p/v %,
dans lequel la quantité d'adsorption d'albumine sérique bovine par 1 mg des particules inorganiques ayant une capacité d'adsorption est de 10 µg ou plus et de 500 µg ou moins.

2. Procédé selon la revendication 1,
dans lequel les particules inorganiques sont des particules composées d'au moins un type de silicate stratifié gonflable choisi dans le groupe constitué par la montmorillonite, l'hectorite, la stevensite, la saponite, la beidellite, la nontrite, la sauconite et le mica gonflable.

3. Procédé selon la revendication 1 ou 2,
dans lequel la cellule animale est une cellule prélevée sur un animal, une cellule obtenue par culture d'une cellule prélevée sur un animal, une cellule obtenue en soumettant une cellule prélevée sur un animal à divers traitements, ou une lignée cellulaire cultivée, et/ou
dans lequel la cellule est une cellule animale de mammifère.

4. Procédé selon l'une quelconque des revendications 1 à 3, dans lequel la cellule animale est cultivée dans une plaque de culture pour cellules en suspension afin de former un sphéroïde.
